# EUROPEAN PATENT SPECIFICATION

(11) **EP 3 689 289 B1**
(45) Date of publication and mention of the grant of the patent: **06.04.2022**
(21) Application number: 19382076.8
(22) Date of filing: 01.02.2019
(51) Int. Cl.: A61C 9/00, A61B 1/24, A61C 19/04, A61B 5/00

(54) **DENTAL IMAGING SYSTEM**
ZAHNBILDGEBUNGSSYSTEM
SYSTÈME D'IMAGERIE DENTAIRE

(43) Date of publication of application: 05.08.2020
(73) Proprietor: Smiletronix Technologies Ltd, Cheadle Hulme SK8 7AN (GB)
(72) Inventor: Faranjani, Yashar, CHEADLE HULME, Cheshire SK8 7AN (GB); Hansen, Edward, SPANISH FORK, UT Utah (US)
(74) Representative: ZBM Patents - Zea, Barlocci & Markvardsen

(56) References cited:
- JP-A- 2012 143 466
- US-A1- 2010 020 165
- US-A1- 2014 132 747
- US-B1- 9 131 909

## Description

The disclosure relates to dental imaging systems for imaging a dental arrangement including an upper teeth arch and a lower teeth arch in an oral cavity.

### BACKGROUND

Odontology is a medical field that is constantly evolving. Things that were done several years ago are no longer the same. This is because science and technology are advancing very fast. Many dental tools may be found in the field of odontology. Said tools may comprise imaging tools that may be based on e.g. x-ray, dental CT, photography, etc.

Dental imaging may assist the dentist or patient in diagnosis activities. Many times, dental photography permits identifying things that at first sight have been overlooked. For this reason, dental photography is very important and companies are increasingly designing and producing more and more products for this cause.

With dental photography, dental treatments can be tracked very well along the whole cycle of the treatment. Dental images are already part of the patient's medical history, and said photos may help dentists or patients in tracking activities, such as e.g. to be aware of the particular situation of each patient at any time, whenever required.

Dental photography may also help dentists or patients in the planning of cases such as e.g. dental treatments, oral hygiene planning and improvement, etc. Nowadays, suitably planning preventative measures and dental procedures is very important, since good planning may ensure the success of dental treatments.

In some countries, such as e.g. in the United States, dental photography is used as a legal tool that may be very important. Dental photography may permit keeping certain personal registers, files that cannot be modified and that, therefore, may be used as a true proof or the like.

The patent document KR20150021310A discloses a prior art dental imaging system which is illustrated in figure 1. This prior art system corresponds to a dental tray 10 comprising a front side wall 11 and a rear side wall 12. The front and rear side walls 11, 12 are formed so that the tray 10 for dental photography surrounds the oral dentition. Multiple camera modules 14 are installed at the front side wall 11 and the rear side wall 12. The front side wall 11 and rear side wall 12 are connected to a u-shaped supporting plate 13. The multiple camera modules 14 comprise a light source 15 and an image sensor 16.

US 2010/020165 A1 discloses a dental imaging system for imaging a dental arrangement including an upper teeth arch or a lower teeth arch in an oral cavity of a patient, but does not disclose any plurality of outer cameras focussing on a front side of both the upper and lower teeth arches.

An object of the present disclosure is to provide new dental imaging systems that improve the prior systems aimed at imaging a dental arrangement inside an oral cavity.

### SUMMARY

In an aspect, a dental imaging system is provided for imaging a dental arrangement including an upper teeth arch and a lower teeth arch in an oral cavity of a patient. The dental imaging system includes an outer arch-shaped structure and an inner centralizer structure coupled to each other, and is installable inside the oral cavity in a predefined position with the upper and lower teeth between the outer arch-shaped structure and a camera hub or camera concentrator of the inner centralizer structure.

The outer arch-shaped structure has a plurality of outer cameras arranged on a side of the outer arch-shaped structure in such a way that, when the dental imaging system is installed in the predefined position, the outer cameras focus on a front side of both the upper and lower teeth arches.

The inner centralizer structure has a support structure and the camera hub, the support structure supporting the camera hub which includes a plurality of inner cameras arranged in such a way that, when the dental imaging system is installed in the predefined position, the inner cameras focus on a back side of both the upper and lower teeth arches.

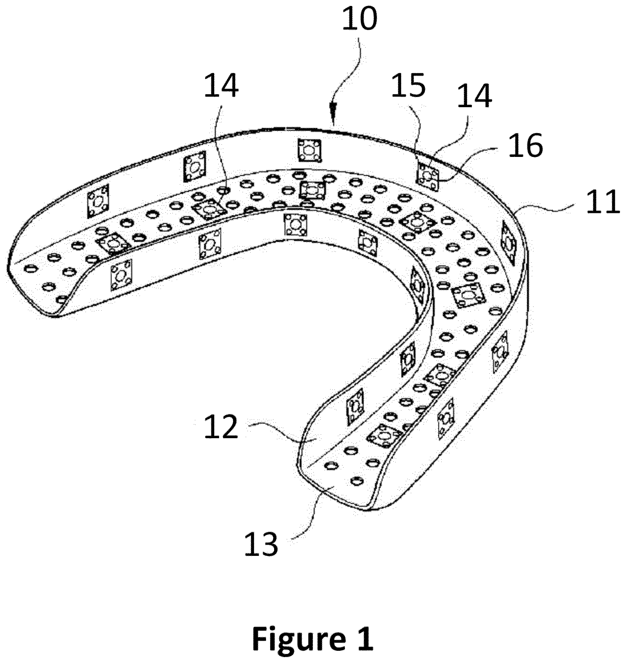

An advantage of the proposed dental imaging system is that fewer cameras may be needed in comparison with prior art dental imaging systems such as the one of figure 1. In the proposed dental imaging system, the inner cameras are centralized in a camera hub instead of being distributed along an inner arch-shaped structure such as the inner structure 12 of figure 1. The camera hub may have any shape suitable for concentrating the inner cameras as required. The camera hub may have a form of e.g. polyhedron, parallelepiped, sphere, etc. or any combination of them, including flat sides, curved sides, irregular shaped sides, etc. or any combination of them.

Moreover, the prior art system of figure 1 is configured to accommodate only one of the teeth arches between outer and inner arch-shaped structures 11, 12. Conversely, the suggested dental imaging system is configured to simultaneously accommodate both upper and lower teeth arches between the outer arch-shaped structure and the inner camera hub or centralizer. This implies that both teeth arches may be simultaneously imaged by the proposed dental imaging system, whereas the prior art system of figure 1 requires a multiple-step operation including imaging one of the two teeth arches firstly and the other one secondly. Such a one-step operation of the dental imaging system disclosed herein versus the prior art multiple-step operation necessarily implies that the proposed dental imaging system is operationally more efficient than prior art dental imaging systems.

According to the above considerations, the dental imaging system proposed herein may be cheaper (less cameras) and operationally more efficient (one-step operation) in comparison with prior art systems.

In some examples, the support structure may be configured (e.g. sized, arranged, etc.) in such a way that, when the dental imaging system is installed in the predefined position, the camera hub occupies a substantially central position in the oral cavity or a radially centred location with respect to both upper and lower teeth arches. This central or centred position may be optimal for the inner cameras (at the camera hub) to completely or almost completely capture the back side of both teeth arches with fewer cameras in comparison with prior art systems.

In configurations of the dental imaging system, the support structure may include one or more support connectors having a first and a second end, the one or more support connectors being coupled with the outer arch-shaped structure at the first end and with the camera hub at the second end. The support connector(s) may be formed as or by one or more bodies of any geometrical shape, such as e.g. any polyhedral (elongated) form suitable to implement the holding function of the support connector(s). In some examples, the support connector(s) may have a shape of rod or bar or the like.

In some implementations, the dental imaging system may comprise one or more stopper pieces configured (e.g. arranged, sized, etc.) to guide the installation of the dental imaging system in the predefined position, in which the front side of both the upper and lower teeth arches may be stopped and, therefore, kept by the stopper piece(s) at a predefined distance from the outer cameras.

Such stopper piece(s) may be seen or understood as separator or spacer piece(s) between the outer cameras and the upper and lower teeth arches, so as to keep or retain the front side of both teeth arches at a predetermined distance from the outer cameras. This predefined distance may depend on the technical features of the outer cameras, their distribution along the outer arch-shaped structure, etc. This predetermined distance may be defined longer or shorter depending on the aforementioned aspects of the outer cameras, and the stopper piece(s) may be configured (i.e. shaped, arranged, etc.) accordingly.

Stopper piece(s) may be comprised in the outer arch-shaped structure, in which case stopper piece(s) may be formed as protuberances arranged on same side of the outer arch-shaped structure where the outer cameras are disposed. Additionally or alternatively, stopper piece(s) may be or may comprise protuberances arranged at e.g. an end of the support connector(s) at or near the coupling between the outer arch-shaped structure and the support connector(s).

According to some examples, the dental imaging system may comprise one or more teether pieces configured (e.g. arranged, sized, etc.) to guide the installation of the dental imaging system in the predefined position, which may include the teether piece(s) bitten by the patient and, therefore, arranged or stuck or sandwiched between the upper teeth and the lower teeth of the patient. Taking this into account, the predefined (installation) position may be also denominated as predefined bite position.

Teether piece(s) may be comprised in the outer arch-shaped structure, in which case teether piece(s) may be formed as teether portions protruding from same side of the outer arch-shaped structure where the outer cameras are disposed. Additionally or alternatively, teether piece(s) may be comprised in the inner centralizer structure, in which case teether piece(s) may be or may be comprised in the support connector(s).

Teether piece(s) may be arranged at same or similar height as the outer cameras or, in other words, may be substantially levelled or aligned with respect to the outer cameras. This manner, the outer cameras may result substantially centred or levelled or aligned or equally distanced with respect to the upper teeth arch and to the lower teeth arch, when the dental imaging system is in predefined (bite) position.

In configurations of the dental imaging system, the outer cameras may be arranged in such a way that, when the dental imaging system is installed in the predefined position, respective individual focus scopes of the outer cameras jointly or cooperatively form an overall focus scope completely or almost completely covering the front side of both the upper and lower teeth arches. This technical feature may be implemented by taking into account the technical peculiarities of cameras to be used as outer cameras. Any skilled person should be able to determine quantity, distribution, arrangement, etc. of the outer cameras in order to provide said "outer" overall focus scope depending on their technical characteristics.

In examples of the dental imaging system, the inner cameras may be arranged or centralized in the camera hub in such a way that, when the dental imaging system is in the predefined position, respective individual focus scopes of the inner cameras jointly or cooperatively form an overall focus scope completely or almost completely covering the back side of both the upper and lower teeth arches. This feature may be implemented by taking into account the technical peculiarities of the cameras to be used as inner cameras. Any skilled person should be able to determine quantity, distribution, arrangement, etc. of the inner cameras in order to provide such an overall focus scope depending on their technical characteristics.

According to configurations of the dental imaging system, the outer arch-shaped structure may have an outwardly protruding shape to push lips away from dental imaging zone and, therefore, avoiding lip interferences or disturbances during dental imaging. This may facilitate the operation of the dental imaging system so as to obtain images of the teeth more easily and with "cleaner" conditions.

In implementations of the dental imaging system, the inner centralizer structure may further include a downwardly and/or backwardly protruding shaped piece to push tongue away from dental imaging zone and, therefore, avoiding tongue interferences or disturbances during dental imaging. This downwardly and/or backwardly protruding shaped piece may be coupled to the camera hub or may be a part of the camera hub itself. This feature may also permit facilitating the operation of the dental imaging system to obtain images of the teeth more easily and under less- or non-interfering conditions.

The dental imaging system may be, in some implementations, formed according to a detachable or removable approach such that any component of the dental imaging system is replaceable for hygiene purposes or the like.

The dental imaging system may comprise, in different examples, a battery for powering the inner and outer cameras. This battery may be a rechargeable battery and, in some examples, the dental imaging system may further comprise a wireless charging system for charging the rechargeable battery. This wireless charging system may be e.g. compatible or operable with a pre-existing electric charger, such as e.g. a toothbrush charger.

The dental imaging system may comprise, in various examples, a connection module to connect the dental imaging system with a computing device. This way, images obtained by the dental imaging system are transmittable to the computing device for processing and/or analysis purposes or the like. This connection module may be configured to implement a wireless connection between the dental imaging system and the computing device.

The dental imaging system may also comprise, in diverse implementations, a lighting system configured to provide light in different visible spectrum and/or invisible spectrum wavelengths, so as to capture dental images under different lighting conditions.

The dental imaging system may comprise, in various configurations, a size-adjusting mechanism associated with the outer arch-shaped structure and/or the inner centralizer structure for regulating their size depending on the patient into whom the dental imaging system is to be applied or installed.

In implementations of the dental imaging system, the outer and inner cameras may be arranged in such a way that, when the dental imaging system is installed with the patient's jaw widened, respective individual focus scopes of the outer and inner cameras jointly or cooperatively form an overall focus scope completely or almost completely covering a top side of both the upper and lower teeth.

### BRIEF DESCRIPTION OF THE DRAWINGS

Non-limiting examples of the disclosure will be described in the following, with reference to the appended drawings, in which:
Figure 1 is a schematic perspective view of a prior art dental imaging system; and
Figure 2 is a schematic perspective view of an imaging system according to examples.

### DETAILED DESCRIPTION OF EXAMPLES

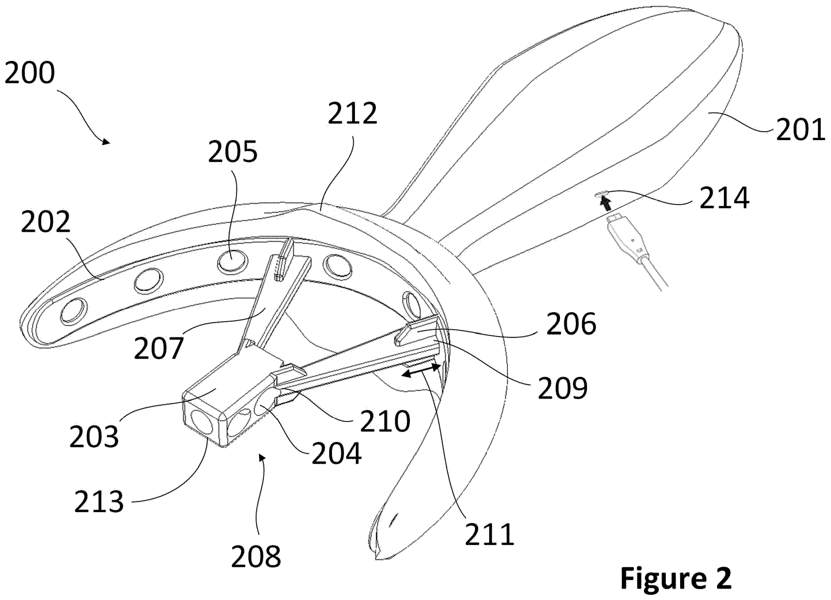

Figure 2 is a schematic perspective view of imaging systems according to examples. Such dental imaging systems 200 may be used for imaging a dental arrangement including an upper teeth arch and a lower teeth arch in an oral cavity. Dental imaging systems 200 may include an outer arch-shaped structure 202 and an inner centralizer structure 208 coupled to each other. Such dental imaging systems 200 may be installable inside the oral cavity in a predefined position with the upper and lower teeth disposed between the outer arch-shaped structure 202 and an inner camera hub 203 of the inner centralizer structure 208.

The outer arch-shaped structure 202 may have outer cameras 205 arranged on a side of the outer arch-shaped structure 202 in such a way that, when the dental imaging system 200 is installed in the predefined position, the outer cameras 205 focus on a front side of both the upper and lower teeth arch.

The inner centralizer structure 208 may include a support structure 207 and the inner camera hub 203. The support structure 207 may hold or support the inner camera hub 203. The inner camera hub 203 may include a plurality of inner cameras 204 arranged in such a way that, when the dental imaging system 200 is installed in the predefined position, the inner cameras 204 focus on a back side of the upper and lower teeth arch.

The support structure may include one or more support connectors 207 having a first end 209 and a second end 210. The one or more support connectors 207 may be coupled with the outer arch-shaped structure 202 at the first end 209 and with the inner camera hub 203 at the second end 210.

The outer arch-shaped structure 202, when installed in the predefined position, may longitudinally and/or externally surround the patient's upper and lower teeth arches. The outer cameras 205 may be distributed regularly or uniformly or equidistantly along corresponding side of the outer arch-shaped structure 202, so as to jointly or cooperatively focus on a whole or almost whole front side of both the upper and lower teeth arches.

The inner camera hub 203 may be or may be formed as a centralizer or concentrator of the inner cameras 204. The inner camera hub 203, when installed in the predefined position, may occupy a substantially central position in the oral cavity and/or with respect to the (patient's) upper and lower teeth arches. This central position may be e.g. a substantially radially centred position with respect to the upper and lower teeth arches. The inner cameras 204 may be centralized, arranged in the inner camera hub 203 so as to jointly or cooperatively focus on a whole or almost whole back side of the upper and lower teeth arches.

The proposed dental imaging systems 200 may include guiding element(s) 206, 207 to permit or facilitate the installation in a guided manner of the dental imaging system 200 in the predefined position inside patient's oral cavity. Such guiding element(s) may comprise e.g. one or more stopper pieces 206 and/or one or more teether pieces 207.

The one or more stopper pieces 206 may be configured (e.g. arranged, sized, etc.) to guide or assist in the installation of the dental imaging system 200 in the predefined position. Stopper piece(s) 206 may be or may act as a separator or spacer between the outer cameras 205 and the upper and lower teeth arches, in the sense that the outer cameras 205 are kept or retained at a predefined distance 211 from the front side of the upper and lower teeth.

The stopper piece(s) 206 may be comprised in the outer arch-shaped structure 202 and/or the inner centralizer structure 208. In the former case, stopper piece(s) 206 may be formed as protuberances arranged on same side of the outer arch-shaped structure 202 where the outer cameras 205 are disposed. In the latter case, stopper piece(s) 206 may be formed as protuberances arranged at e.g. the first end 209 of the support connector(s) 207, i.e. at or near the coupling 209 between the outer arch-shaped structure 202 and the support connector(s) 207.

The one or more teether pieces 207 may be configured (e.g. arranged, sized, etc.) to also guide or assist in the installation of the dental imaging system 200 in the predefined position. The teether piece(s) 207 may be configured to be bitten by the patient and, therefore, to be arranged or stuck or sandwiched between the upper teeth and the lower teeth. In this sense, the predefined installation position may also be denominated as predefined bite position.

The teether piece(s) 207 may be comprised in the outer arch-shaped structure 202 and/or in the inner centralizer structure 208. In the former case, teether piece(s) 207 may be formed as teether portions protruding from same side of the outer arch-shaped structure 202 where the outer cameras 205 are disposed. In the latter case, teether piece(s) 207 may be or may be comprised in the support connector(s) 207 of the inner centralizer structure 208.

The teether piece(s) 207 may be arranged at same or similar height as the outer cameras 205 or, in other words, the teether piece(s) 207 may be substantially levelled or aligned with respect to the outer cameras 205. This way, when the dental imaging system 200 is in the bite position, the outer cameras 205 may result substantially centred or levelled or aligned or equally distanced with respect to the upper teeth arch and to the lower teeth arch.

The inner centralizer structure 208 may be configured (e.g. sized, arranged, etc.) in such a way that, when the dental imaging system 200 is installed in the predefined position, the inner camera hub 203 results positioned at a substantially central position of the oral cavity.

The outer cameras 205 may be arranged in such a way that, when the dental imaging system 200 is installed in the predefined position, respective individual focus scopes of the outer cameras 205 jointly form an overall focus scope completely or almost completely covering the front side of both the upper and lower teeth arch.

The inner cameras 204 may be arranged, at the inner camera hub 203, in such a way that, when the dental imaging system 200 is installed in the predefined position, respective individual focus scopes of the inner cameras 204 jointly form an overall focus scope completely or almost completely covering the back side of both the upper and lower teeth arch.

Dental imaging system 200 may comprise a lighting system (not shown) configured to provide light in different visible spectrum and/or invisible spectrum wavelengths, so as to allow capturing images under different lighting conditions, depending on e.g. circumstances, type of photos to be produced, purpose of the photos, diagnosis of different conditions, etc. This lighting system may comprise different light sources suitably configured (arranged, sized, etc.). Any relation between light sources and cameras 204, 205 may exist, such as e.g. one-to-one relation, one-to-many, etc.

The outer arch-shaped structure 202 may have an outwardly protruding shape 212 to push lips away from dental imaging zone and, therefore, avoiding lip interferences or disturbances during dental imaging.

The inner centralizer structure 208 may further include a downwardly and/or backwardly protruding shaped piece 213 to push tongue away from dental imaging zone and, therefore, avoiding tongue interferences or disturbances during dental imaging. The downwardly and/or backwardly protruding shaped piece 213 may be coupled to the inner camera hub 203 or may be comprised in the inner camera hub itself 203.

Dental imaging systems 200 according to present disclosure may be formed according to a detachable approach such that any component of the dental imaging system 200 may be replaceable for e.g. hygiene purposes, customizing aims, repairability, etc.

Dental imaging systems 200 may comprise a connection module 214 to connect the dental imaging system 200 with a computing device, so that images obtained by the dental imaging system 200 are transmittable to the computing device for e.g. processing data, image transfer, analysis, diagnosis, and/or recommendation purposes or the like. The connection module may be configured to implement a wireless connection between the dental imaging system 200 and the computing device. Some computing functions may be part of or may be comprised in the connection module 214.

Dental imaging systems 200 may comprise one or more batteries for powering the inner and outer cameras 204, 205, lighting system, connection module 214, etc. The one or more batteries may be rechargeable. Dental imaging systems 200 may further comprise a wireless charging system for charging the one or more rechargeable batteries. The wireless charging system may be compatible with pre-existing electric charger(s), such as e.g. pre-existing electric toothbrush charger(s).

The suggested dental imaging systems 200 may further comprise a handle 201 or similar piece for easy, comfortable, protective and/or efficient manipulation of the dental imaging system 200. The aforementioned connection module 214 may be comprised in said handle 201.

In some examples, dental imaging systems 200 may comprise a size-adjusting mechanism associated with the outer arch-shaped structure 202, the inner centralizer structure 208, etc. for regulating the size of such components depending on each patient into whom the dental imaging system 200 is to be applied or installed. This way, a same dental imaging system 200 may be used with different patients having differently sized oral cavities, teeth arches, etc.

Any of the dental imaging systems 200 described herein may be used to image or photograph teeth with the purpose of assisting individuals and/or dentists in achieving dental diagnosis. The obtained images may be inspected and/or analysed by using systems based on artificial intelligence (Al), machine learning (ML) approaches, or even through visual inspection by a human being properly skilled for that aim. These inspection and/or analysis functions may be performed by computing system(s) comprised in the dental imaging system 200, or by computing system(s) outside the imaging system 200 connectable with each other.

Dental imaging systems 200 may comprise a connection with a recommendation engine configured to produce recommendations about dental treatments, oral hygiene practices, dietary habits, other oral or non-oral health conditions, etc. on users or patients. Alternatively, the recommendation engine may be comprised in the dental imaging system 200. A reporting system may be associated to or comprised in the recommendation engine so as to send messages or notifications to users about said recommendations. This messaging may be performed through e.g. a suitable messaging device or platform associated with or comprised in the dental imaging system 200.

Dental imaging systems 200 according to present disclosure may thus be or may constitute or form (along with e.g. Al-based system, ML-based system, recommendation engine, etc.) a smart system including e.g. self-diagnosis functionalities, self-planning functionalities, self-tracking functionalities, etc.

In some configurations, the arrangement of the outer cameras 205 in/on the outer arch-shaped structure 202 and of the inner cameras 204 in/on the inner camera hub 203 may be such that, when the dental imaging system 200 is installed with patient's jaw widened, a top side of the upper and lower teeth may be imaged by the cameras 204, 205. That is, with such an arrangement, respective individual vision or focus scopes of the inner and outer cameras 204, 205 may jointly or cooperatively form an overall focus scope completely or almost completely covering a top side of both the upper and lower teeth. In some examples, the position of the inner and outer cameras 204, 205 may be adjustable in order to properly move their individual focus scopes. Any position-regulating mechanism may be used to implement said adjustability with one or more degrees of freedom, such as e.g. upwards, downwards, leftwards, rightwards, etc.

The scope of the disclosure should not be limited by particular examples, but should be determined only by the claims that follow.

## Claims

1. A dental imaging system (200) for imaging a dental arrangement including an upper teeth arch and a lower teeth arch in an oral cavity of a patient, the dental imaging system (200) including an outer arch-shaped structure (202) and an inner centralizer structure (208) coupled to each other, and being installable inside the oral cavity in a predefined position with the upper and lower teeth between the outer arch-shaped structure (202) and a camera hub (203) of the inner centralizer structure (208), and wherein
the outer arch-shaped structure (202) has a plurality of outer cameras (205) arranged on a side of the outer arch-shaped structure (202) in such a way that, when the dental imaging system (200) is installed in the predefined position, the outer cameras (205) focus on a front side of both the upper and lower teeth arches; and
the inner centralizer structure (208) includes the camera hub (203) and a support structure (207) holding the camera hub (203), and the camera hub (203) including a plurality of inner cameras (204) arranged in such a way that, when the dental imaging system (200) is installed in the predefined position, the inner cameras (204) focus on a back side of both the upper and lower teeth arches.

2. A dental imaging system (200) according to claim 1, wherein the support structure (207) is configured in such a way that, when the dental imaging system (200) is installed in the predefined position, the inner camera hub (203) is disposed at a substantially central position of the oral cavity.

3. A dental imaging system (200) according to any of claims 1 or 2, wherein the support structure (207) includes one or more support connectors (207) having a first end (209) and a second end (210), the one or more support connectors (207) being coupled with the outer arch-shaped structure (202) at the first end (209) and with the inner camera hub (203) at the second end (210).

4. A dental imaging system (200) according to any of claims 1 to 3, comprising one or more stopper pieces (206) configured to guide the installation of the dental imaging system (200) in the predefined position, in which the front side of both the upper and lower teeth is kept or retained by the one or more stopper pieces (206) at a predefined distance (211) from the outer cameras (205).

5. A dental imaging system (200) according to any of claims 1 to 4, comprising one or more teether pieces (207) configured to guide the installation of the dental imaging system (200) in the predefined position, in which the one or more teether pieces (207) are bitten by the patient and therefore arranged or stuck or sandwiched between the upper teeth and the lower teeth of the patient.

6. A dental imaging system (200) according to any of claims 1 to 5, wherein the outer cameras (205) are arranged in such a way that, when the dental imaging system (200) is installed in the predefined position, respective individual focus scopes of the outer cameras (205) jointly form an overall focus scope completely or almost completely covering the front side of both the upper and lower teeth arches.

7. A dental imaging system (200) according to any of claims 1 to 6, wherein the inner cameras (204) are arranged in such a way that, when the dental imaging system (200) is installed in the predefined position, respective individual focus scopes of the inner cameras (204) jointly form an overall focus scope completely or almost completely covering the back side of both the upper and lower teeth arches.

8. A dental imaging system (200) according to any of claims 1 to 7, wherein the outer arch-shaped structure (202) has an outwardly protruding shape (212) to push lips away from dental imaging zone and, therefore, avoiding lip interferences during dental imaging.

9. A dental imaging system (200) according to any of claims 1 to 8, wherein the inner centralizer structure (208) further includes a downwardly and/or backwardly protruding shaped piece (213) to push tongue away from dental imaging zone and, therefore, avoiding tongue interferences during dental imaging; wherein the downwardly and/or backwardly protruding shaped piece (213) is coupled to the inner camera hub (203) or is comprised in the inner camera hub (203) itself.

10. A dental imaging system (200) according to any of claims 1 to 9, formed according to a detachable or removable approach such that any component of the dental imaging system (200) is replaceable.

11. A dental imaging system (200) according to any of claims 1 to 10, comprising a battery for powering the inner and outer cameras (205) or any other electrical component in the dental imaging system (200); wherein the battery is a rechargeable battery; and wherein the dental imaging system (200) further comprises a wireless charging system for charging the rechargeable battery.

12. A dental imaging system (200) according to any of claims 1 to 11, comprising a connection module (214) to connect the dental imaging system (200) with a computing device, so that images obtained by the dental imaging system (200) are transmittable to the computing device; wherein the connection module (214) is configured to implement a wireless connection between the dental imaging system (200) and the computing device.

13. A dental imaging system (200) according to any of claims 1 to 12, comprising a lighting system configured to provide light in different visible spectrum and/or invisible spectrum wavelengths, so as to capture dental images under different lighting conditions.

14. A dental imaging system (200) according to any of claims 1 to 13, comprising a size-adjusting mechanism associated with the outer arch-shaped structure (202) and/or the inner centralizer structure (208) for regulating their size depending on the patient into whom the dental imaging system (200) is to be applied or installed.

## Patentansprüche

1. Ein dentales Bildgebungssystem (200) zur Bildgebung einer Zahnanordnung mit einem oberen Zahnbogen und einem unteren Zahnbogen in einer Mundhöhle eines Patienten, wobei das dentale Bildgebungssystem (200) eine äußere bogenförmige Struktur (202) und eine innere Zentrierstruktur (208) umfasst, die miteinander gekoppelt sind, und innerhalb der Mundhöhle in einer vordefinierten Position installierbar ist, wobei die oberen und unteren Zähne zwischen der äußeren bogenförmigen Struktur (202) und einer Kameranabe (203) der inneren Zentrierstruktur (208) angeordnet sind, und wobei
die äußere bogenförmige Struktur (202) eine Vielzahl von äußeren Kameras (205) aufweist, die an einer Seite von der äußeren bogenförmigen Struktur (202) derart angeordnet sind, dass, wenn das dentale Bildgebungssystem (200) in der vordefinierten Position installiert ist, die äußeren Kameras (205) auf eine Vorderseite sowohl des oberen als auch des unteren Zahnbogens fokussieren; und
die innere Zentrierstruktur (208) die Kameranabe (203) und eine die Kameranabe (203) haltende Stützstruktur (207) umfasst, und wobei die Kameranabe (203) eine Vielzahl von inneren Kameras (204) umfasst, die so angeordnet sind, dass die inneren Kameras (204) auf einer Rückseite sowohl des oberen als auch des unteren Zahnbogens fokussieren, wenn das dentale Bildgebungssystem (200) in der vordefinierten Position installiert ist.

2. Ein dentales Bildgebungssystem (200) nach Anspruch 1, wobei die Stützstruktur (207) derart konfiguriert ist, dass, wenn das dentale Bildgebungssystem (200) in der vordefinierten Position installiert ist, die Nabe (203) der inneren Kameras an einer im Wesentlichen zentralen Position der Mundhöhle angeordnet ist.

3. Ein dentales Bildgebungssystem (200) nach einem der Ansprüche 1 oder 2, wobei die Stützstruktur (207) einen oder mehrere Stützverbinder (207) mit einem ersten (209) und einem zweiten Ende (210) umfasst, wobei einer oder mehrere Stützverbinder (207) am ersten Ende (209) mit der äußeren bogenförmigen Struktur (202) und am zweiten Ende (210) mit der Nabe (203) der inneren Kameras gekoppelt sind.

4. Ein dentales Bildgebungssystem (200) nach einem der Ansprüche 1 bis 3, umfassend ein oder mehrere Anschlagstücke (206), die konfiguriert sind, um die Installation des dentalen Bildgebungssystems (200) in der vordefinierten Position zu führen, in der die Vorderseite sowohl der oberen als auch der unteren Zähne durch das eine oder die mehreren Anschlagstücke (206) in einem vordefinierten Abstand (211) von den äußeren Kameras (205) gehalten oder zurückgehalten wird.

5. Ein dentales Bildgebungssystem (200) nach einem der Ansprüche 1 bis 4, mit einem oder mehreren Beißringstücken (207), die konfiguriert sind, um die Installation des dentalen Bildgebungssystems (200) in der vordefinierten Position zu führen, in der das eine oder die mehreren Beißringstücke (207) vom Patienten gebissen und daher angeordnet werden oder zwischen den oberen Zähnen und den unteren Zähnen des Patienten festgeklemmt oder eingeklemmt werden.

6. Ein dentales Bildgebungssystem (200) nach einem der Ansprüche 1 bis 5, wobei die äußeren Kameras (205) so angeordnet sind, dass bei einer Installation des dentalen Bildgebungssystems (200) in der vordefinierten Position jeweilige einzelne Fokusbereiche der äußeren Kameras (205) gemeinsam einen Gesamtfokusbereich bilden, der die Vorderseite sowohl des oberen als auch des unteren Zahnbogens vollständig oder fast vollständig abdeckt.

7. Ein dentales Bildgebungssystem (200) nach einem der Ansprüche 1 bis 6, wobei die inneren Kameras (204) so angeordnet sind, dass bei einer Installation des dentalen Bildgebungssystems (200) in der vordefinierten Position jeweilige einzelne Fokusbereiche der inneren Kameras (204) gemeinsam einen Gesamtfokusbereich bilden, der die Hinterseite sowohl des oberen als auch des unteren Zahnbogens vollständig oder fast vollständig abdeckt.

8. Ein dentales Bildgebungssystem (200) nach einem der Ansprüche 1 bis 7, wobei die äußere bogenförmige Struktur (202) eine nach außen vorstehende Form (212) hat, um Lippen von der dentalen Bildgebungszone wegzudrücken und daher Lippeninterferenzen während der dentalen Bildgebung zu vermeiden.

9. Ein dentales Bildgebungssystem (200) nach einem der Ansprüche 1 bis 8, wobei die innere Zentrierstruktur (208) ferner ein nach unten und/oder nach hinten vorstehendes Formstück (213) umfasst, um die Zunge von der dentalen Bildgebungszone wegzudrücken und daher Zungeninterferenzen während der dentalen Bildgebung zu vermeiden; wobei das nach unten und/oder nach hinten vorstehende Formstück (213) mit der Nabe (203) der inneren Kameras gekoppelt ist oder in der Nabe (203) der inneren Kameras selbst enthalten ist.

10. Ein dentales Bildgebungssystem (200) nach einem der Ansprüche 1 bis 9, das gemäß einem abnehmbaren oder entfernbaren Ansatz ausgebildet ist, so dass jede Komponente des dentalen Bildgebungssystems (200) austauschbar ist.

11. Ein dentales Bildgebungssystem (200) nach einem der Ansprüche 1 bis 10, umfassend eine Batterie zum Antreiben der inneren und äußeren Kameras (205) oder jeder anderen elektrischen Komponente des dentalen Bildgebungssystems (200); wobei die Batterie eine wiederaufladbare Batterie ist; und wobei das dentale Bildgebungssystem (200) ferner ein drahtloses Ladesystem zum Laden der wiederaufladbaren Batterie umfasst.

12. Ein dentales Bildgebungssystem (200) nach einem der Ansprüche 1 bis 11, umfassend ein Verbindungsmodul (214), um das dentale Bildgebungssystem (200) mit einer Rechenvorrichtung zu verbinden, so dass durch das dentale Bildgebungssystem (200) erhaltene Bilder an die Rechenvorrichtung übertragbar sind; wobei das Verbindungsmodul (214) konfiguriert ist, um eine drahtlose Verbindung zwischen dem dentalen Bildgebungssystem (200) und der Computervorrichtung zu implementieren.

13. Ein dentales Bildgebungssystem (200) nach einem der Ansprüche 1 bis 12, umfassend ein Beleuchtungssystem, das konfiguriert ist, um Licht in verschiedenen Wellenlängen des sichtbaren Spektrums und/oder des unsichtbaren Spektrums bereitzustellen, um so dentale Bilder unter verschiedenen Beleuchtungsbedingungen aufzunehmen.

14. Ein dentales Bildgebungssystem (200) nach einem der Ansprüche 1 bis 13, umfassend einen der äußeren bogenförmigen Struktur (202) und/oder der inneren Zentrierstruktur (208) zugeordneten Größeneinstellmechanismus zum Regulieren ihrer Größe in Abhängigkeit von dem Patienten, in den das dentale Bildgebungssystem (200) aufgebracht oder installiert werden soll.

## Revendications

1. Un système d'imagerie dentaire (200) pour l'imagerie d'un agencement dentaire comprenant une arcade dentaire supérieure et une arcade dentaire inférieure dans une cavité buccale d'un patient, le système d'imagerie dentaire (200) comprenant une structure extérieure en forme d'arc (202) et une structure intérieure de centrage (208) couplées l'une à l'autre, et pouvant être installé à l'intérieur de la cavité buccale dans une position prédéfinie avec les dents supérieures et inférieures entre la structure extérieure en forme d'arc (202) et un moyeu de caméra (203) de la structure intérieure de centrage (208), et dans lequel
la structure extérieure en forme d'arc (202) a une pluralité de caméras extérieures (205) disposées sur un côté de la structure extérieure en forme d'arc (202) de telle manière que, lorsque le système d'imagerie dentaire (200) est installé dans la position prédéfinie, les caméras extérieures (205) mettent au point sur une face avant des arcades dentaires supérieure et inférieure ; et
la structure intérieure de centrage (208) comprend le moyeu de caméra (203) et une structure de support (207) maintenant le moyeu de caméra (203), et le moyeu de caméra (203) comprenant une pluralité de caméras intérieures (204) agencées de telle manière que, lorsque le système d'imagerie dentaire (200) est installé dans la position prédéfinie, les caméras intérieures (204) mettent au point sur une face arrière des arcades dentaires supérieure et inférieure.

2. Un système d'imagerie dentaire (200) selon la revendication 1, dans lequel la structure de support (207) est configurée de telle manière que, lorsque le système d'imagerie dentaire (200) est installé dans la position prédéfinie, le moyeu de caméra intérieure (203) est disposé dans une position essentiellement centrale de la cavité buccale.

3. Un système d'imagerie dentaire (200) selon l'une quelconque des revendications 1 ou 2, dans lequel la structure de support (207) comprend un ou plusieurs connecteurs de support (207) ayant une première (209) et une seconde extrémité (210), le ou les connecteurs de support (207) étant couplés avec la structure extérieure en forme d'arc (202) à la première extrémité (209) et avec le moyeu de caméra intérieure (203) à la deuxième extrémité (210).

4. Un système d'imagerie dentaire (200) selon l'une quelconque des revendications 1 à 3, comprenant une ou plusieurs pièces d'arrêt (206) configurées pour guider l'installation du système d'imagerie dentaire (200) dans la position prédéfinie, dans laquelle la face avant des dents supérieures et inférieures est maintenue ou retenue par la ou les pièces d'arrêt (206) à une distance prédéfinie (211) des caméras extérieures (205).

5. Un système d'imagerie dentaire (200) selon l'une quelconque des revendications 1 à 4, comprenant une ou plusieurs pièces de hochet (207) configurées pour guider l'installation du système d'imagerie dentaire (200) dans la position prédéfinie, dans laquelle la ou les pièces de hochet (207) sont mordues par le patient et donc disposées ou coincées ou prises en sandwich entre les dents supérieures et les dents inférieures du patient.

6. Un système d'imagerie dentaire (200) selon l'une quelconque des revendications 1 à 5, dans lequel les caméras extérieures (205) sont agencées de telle manière que, lorsque le système d'imagerie dentaire (200) est installé dans la position prédéfinie, les cadres de mise au point individuels respectives des caméras extérieures (205) forment conjointement un cadre de mise au point global couvrant complètement ou presque complètement la face avant des arcades dentaires supérieure et inférieure.

7. Un système d'imagerie dentaire (200) selon l'une quelconque des revendications 1 à 6, dans lequel les caméras intérieures (204) sont agencées de telle manière que, lorsque le système d'imagerie dentaire (200) est installé dans la position prédéfinie, les cadres de mise au point individuels respectifs des caméras intérieures (204) forment conjointement un cadre de mise au point global couvrant complètement ou presque complètement la face arrière des arcades dentaires supérieure et inférieure.

8. Un système d'imagerie dentaire (200) selon l'une quelconque des revendications 1 à 7, dans lequel la structure extérieure en forme d'arc (202) a une forme faisant saillie vers l'extérieur (212) pour éloigner les lèvres de la zone d'imagerie dentaire et, par conséquent, éviter les interférences des lèvres pendant l'imagerie dentaire.

9. Un système d'imagerie dentaire (200) selon l'une quelconque des revendications 1 à 8, dans lequel la structure intérieure de centrage (208) comprend en outre une pièce façonnée faisant saillie vers le bas et/ou vers l'arrière (213) pour éloigner la langue de la zone d'imagerie dentaire et, par conséquent, éviter les interférences de la langue pendant l'imagerie dentaire ; dans lequel la pièce façonnée (213) faisant saillie vers le bas et/ou vers l'arrière est couplée au moyeu de caméra (203) intérieure ou est comprise dans le moyeu de caméra (203) intérieure lui-même.

10. Un système d'imagerie dentaire (200) selon l'une quelconque des revendications 1 à 9, formé selon une approche détachable ou amovible de telle sorte que tout composant du système d'imagerie dentaire (200) est remplaçable.

11. Un système d'imagerie dentaire (200) selon l'une quelconque des revendications 1 à 10, comprenant une batterie pour alimenter les caméras intérieure et extérieure (205) ou tout autre composant électrique dans le système d'imagerie dentaire (200) ; dans lequel la batterie est une batterie rechargeable ; et dans lequel le système d'imagerie dentaire (200) comprend en outre un système de charge sans fil pour charger la batterie rechargeable.

12. Un système d'imagerie dentaire (200) selon l'une quelconque des revendications 1 à 11, comprenant un module de connexion (214) pour connecter le système d'imagerie dentaire (200) à un dispositif informatique, de sorte que les images obtenues par le système d'imagerie dentaire (200) sont transmissibles au dispositif informatique ; dans lequel le module de connexion (214) est configuré pour mettre en oeuvre une connexion sans fil entre le système d'imagerie dentaire (200) et le dispositif informatique.

13. Un système d'imagerie dentaire (200) selon l'une quelconque des revendications 1 à 12, comprenant un système d'éclairage configuré pour fournir de la lumière dans de différentes longueurs d'onde du spectre visible et/ou du spectre invisible, de manière à capturer des images dentaires dans de différentes conditions d'éclairage.

14. Un système d'imagerie dentaire (200) selon l'une quelconque des revendications 1 à 13, comprenant un mécanisme de réglage de taille associé à la structure extérieure en forme d'arc (202) et/ou à la structure intérieure de centrage (208) pour régler leur taille en fonction du patient dans lequel le système d'imagerie dentaire (200) doit être appliqué ou installé.
